# EUROPÄISCHE PATENTSCHRIFT

(11) **EP 3 967 458 B1**
(45) Veröffentlichungstag und Bekanntmachung des Hinweises auf die Patenterteilung: **05.07.2023**
(21) Anmeldenummer: 21187370.8
(22) Anmeldetag: 23.07.2021
(51) Int. Cl.: B25J 9/00, A61F 5/28

(54) **VORRICHTUNG ZUR UNTERSTÜTZUNG VON BEWEGUNGEN UND HUBBEWEGUNGEN DES MENSCHLICHEN KÖRPERS**
DEVICE FOR SUPPORTING MOVEMENTS AND LIFTING MOVEMENTS OF THE HUMAN BODY
DISPOSITIF D'AIDE AUX MOUVEMENTS ET AUX MOUVEMENTS DE LEVAGE DU CORPS HUMAIN

(30) Priorität: 14.08.2020 DE 102020121398
(43) Veröffentlichungstag der Anmeldung: 16.03.2022
(73) Patentinhaber: HUNIC GmbH, 72270 Baiersbronn (DE)
(72) Erfinder: Mast, Jonas, 72270 Baiersbronn (DE); Ponzoni Dognini, Eduardo, 70193 Stuttgart (DE)
(74) Vertreter: Westphal, Mussgnug & Partner Patentanwälte mbB

(56) Entgegenhaltungen:
- DE-A1-102017 123 518
- US-A1- 2008 125 685

## Beschreibung

Die Erfindung betrifft eine Vorrichtung zur Unterstützung von Bewegungen und Hubbewegungen des menschlichen Körpers, welche am Körper tragbar ist.

Viele Personen, insbesondere gewerbliche Arbeitnehmer, sind bei unterschiedlichen Tätigkeiten hohen physischen Belastungen ausgesetzt. Hierbei wird insbesondere der Rücken beim Heben und Tragen von Lasten, durch ungünstige Haltungen oder sich ständig wiederholende manuelle Tätigkeiten stark belastet. Um hier eine Unterstützung zu ermöglichen, sind Vorrichtungen zur Unterstützung von Bewegungen und Hubbewegungen des menschlichen Körpers, welche am Körper tragbar sind, bekannt. Derartige Vorrichtungen werden auch als Exoskelette bezeichnet.

Dabei sind aktive und passive Exoskelette bekannt. Aktive Exoskelette zeichnen sich dadurch aus, dass sie elektrische Motoren, hydraulische Komponenten oder ähnliche Systeme aufweisen, die Bewegungen der Gelenke des menschlichen Körpers durch ihren Antrieb aktiv unterstützen können. Die Person erfährt somit eine direkte Unterstützung bei seinen Bewegungen und Tätigkeiten. Aktive Exoskelette weisen aufgrund der Maschinenelemente jedoch ein hohes Eigengewicht auf. Zudem ist die Abstimmung des Antriebs der Maschinenelemente auf den Bewegungsvorgang der Person nicht einfach, da die Maschinenelemente erkennen müssen, wann welcher Bewegungsvorgang durchgeführt wird. Schließlich tragen derartige aktive Exoskelette vom Bauraum her auf und sind unhandlich zu handhaben.

Passive Exoskelette weisen keinen Antrieb auf, sondern speichern potentielle Energie bei einer Flexion des Körpers oder des entsprechenden Gelenks. Bei einer Reflexion wird diese Energie wieder freigegeben und unterstützt die entsprechende Bewegung. Bekannt ist beispielsweise die Verwendung von Federn. Das Eigengewicht von passiven Exoskeletten ist in der Regel geringer als das von aktiven Exoskeletten, und üblicherweise trägt das passive Exoskelett weniger auf als das aktive Exoskelett.

Aus der DE 10 2017 123 518 A1, auf die der Oberbegriff des Anspruchs 1 gestützt ist, ist beispielsweise ein passives Exoskelett mit einem Hüftelement, mit zwei Schulterbändern, mit einem Rückenelement, welches an dem Hüftelement angeordnet ist, und mit zwei Beinbändern, welche derart angeordnet sind, dass sie im an den Körper angelegten Zustand der Vorrichtung am Gesäß des Körpers entlang laufen, bekannt. Bei den bekannten Vorrichtungen werden die Beinbänder mittels eines Fußelements am Fuß befestigt, um die Beinbänder zuverlässig fixieren zu können. Das Anlegen der Fußelemente ist jedoch oft umständlich. Zudem sind die Fußelemente, welche um den Fuß und somit über die Fußsohle laufen können, oft unangenehm zu tragen.

Die Aufgabe der Erfindung besteht darin, eine Vorrichtung zur Unterstützung von Bewegungen und Hubbewegungen des menschlichen Körpers, insbesondere ein passives Exoskelett, derart weiterzubilden, dass die Vorrichtung einfacher anzulegen und vorzugsweise angenehmer zu tragen ist.

Die Aufgabe wird erfindungsgemäß gelöst durch eine Vorrichtung zur Unterstützung von Bewegungen und Hubbewegungen des menschlichen Körpers, insbesondere ein passives Exoskelett, mit den Merkmalen des Patentanspruchs 1.

Vorteilhafte Ausgestaltungen und Weiterbildungen der Erfindung sind in den abhängigen Ansprüchen angegeben.

Die erfindungsgemäße Vorrichtung zur Unterstützung von Bewegungen und Hebebewegungen des menschlichen Körpers, welche am Körper tragbar ist, mit einem Hüftelement und mit zwei an dem Hüftelement angeordneten Beinbändern, welche derart angeordnet sind, dass sie im an den Körper angelegten Zustand der Vorrichtung am Gesäß des Körpers entlang verlaufen, wobei jedes der Beinbänder ein Knieelement aufweist, welches eine obere Schlaufe, eine untere Schlaufe und ein die Kniescheibe freilassende Kniescheibenelement aufweist, zeichnet sich dadurch aus, dass der Abstand zwischen der oberen Schlaufe und der unteren Schlaufe verstellbar ausgebildet ist.

Als Hüftelement soll im Folgenden ein Element verstanden werden, das im an eine Person angelegten Zustand der Vorrichtung im Bereich der Hüfte an der Person anliegt. Es kann, muss aber nicht zwingenderweise, zur Befestigung der Vorrichtung an der Person ausgelegt sein. Das Hüftelement kann beispielsweise als Hüftgurt ausgebildet sein, wodurch eine zuverlässige Befestigung der Vorrichtung an einer Person ermöglicht werden kann. Der Hüftgurt ist vorzugsweise in der Weite verstellbar, so dass eine Anpassung an den Hüftumfang der Person, welche die Vorrichtung anlegt, erfolgen kann.

Die Verstellbarkeit des Abstands zwischen der oberen Schlaufe und der unteren Schlaufe ermöglicht eine verbesserte Anpassung der Vorrichtung an die Anatomie des Benutzers. Insbesondere ermöglicht dies ein Anlegen der unteren Schlaufe unterhalb der Wade des Benutzers, so dass ein Fußelement überflüssig wird, da eine zuverlässige Fixierung des Beinbands in Längsrichtung des Beins unterhalb der Wade erfolgen kann. Bei Fixierung der unteren Schlaufe unterhalb der Wade kann das Anlegen der Beinbänder einfacher erfolgen, als wenn das Beinband mit einem Fußelement am Fuß fixiert werden müsste. Außerdem ist eine unterhalb der Wade angelegte untere Schlaufe in der Regel angenehmer zu tragen als ein um die Fußsohle angelegtes Fußelement.

Vorzugsweise ist die untere Schlaufe lösbar an dem Kniescheibenelement befestigbar. Auf diese Weise kann einfach eine Verstellbarkeit des Abstands zwischen der oberen Schlaufe und der unteren Schlaufe ermöglicht werden und zudem das Anlegen der Vorrichtung vereinfach werden.

Besonders bevorzugt ist die untere Schlaufe in unterschiedlichen Positionen an dem Kniescheibenelement fixierbar. Dies ermöglicht auf einfache Art und Weise die Anpassung des Abstands zwischen der oberen Schlaufe und der unteren Schlaufe auf die Länge der Wade eines Benutzers.

Vorteilhafterweise ist die untere Schlaufe mittels eines auf einer Klett- oder Magnetverbindung oder einer formschlüssigen Verbindung basierenden Verschlusses an dem Kniescheibenelement fixierbar, was eine besonders einfache Handhabung ermöglichen kann.

Vorzugsweise ist die untere Schlaufe mittels eines Schienensystems verschiebbar und in unterschiedlichen Positionen fixierbar an dem Kniescheibenelement angeordnet. Dadurch kann eine zuverlässige und genaue Positionierung der unteren Schlaufe ermöglicht werden.

Eine besonders vorteilhafte Weiterbildung der Erfindung sieht vor, dass die obere Schlaufe und/oder die untere Schlaufe mittels eines Verschlusses öffenbar und wiederverschließbar, vorzugsweise mittels eines Klettverschlusses, eines auf einem Formschluss basierenden Verschlusses, eines Magnetverschlusses oder eines sonstigen Verschlusses, ausgebildet sind. Dies vereinfacht das Anlegen des Knieelements, da ein Überstreifen der Schlaufe ausgehend vom Fuß bis zum Kniebereich vermieden werden kann.

Vorteilhafterweise ist an dem Kniescheibenelement ein Befestigungselement angeordnet, welches insbesondere oberhalb der Wade um das Bein angelegt werden kann, wodurch die Fixierung am Körper weiter verbessert werden kann.

Vorzugsweise sind die Beinbänder aus einem Textilmaterial gefertigt.

Alternativ oder zusätzlich sind die Beinbänder vorzugsweise zumindest abschnittsweise aus einem Elastomer gefertigt.

Gemäß einer vorteilhaften Ausgestaltung der Erfindung weist das Elastomer ein E-Modul von weniger als 600 N/mm², beispielsweise von weniger als 150 N/mm², insbesondere von weniger als 100 N/mm², beispielsweise ein E-Modul von etwa 30 N/mm², auf. Dies ermöglicht ausreichend Energie bei der Flexion aufzunehmen und bei der Reflexion ausreichende Unterstützung bieten zu können.

Vorteilhafterweise ist jedes der Beinbänder lösbar an dem Hüftelement befestigt. Dies ermöglicht ein einfacheres Anlegen der Vorrichtung.

Gemäß einer bevorzugten Weiterbildung der Erfindung ist jedes der Beinbänder an der Außenseite des Hüftelements befestigbar, beispielsweise mittels eines Hakens, einer Schnalle, einer Gürtelschnalle oder eines Klettverschlusses. Dies kann das Anlegen und insbesondere Positionieren der Beinbänder besonders einfach gestalten.

Zur verbesserten Führung der Beinbänder ist vorzugweise vorgesehen, dass jedes der Beinbänder mit einem freien Ende durch zwei im Winkel, vorzugsweise im Winkel von 90°, zueinander verlaufende Schlitze, geführt ist.

Vorzugsweise ist jedes der Beinbänder im Bereich zwischen dem Hüftelement und dem Knieelement in der Länge verstellbar ausgebildet, wodurch die Vorrichtung auf einfache Art und Weise an die Größe der Person, die die Vorrichtung anlegt, angepasst werden kann.

Die Erfindung wird anhand der nachfolgenden Figuren ausführlich erläutert. Es zeigen:
- Fig. 1: eine Ansicht von vorne auf ein erstes Ausführungsbeispiel einer erfindungsgemäßen Vorrichtung,
- Fig. 2: eine Ansicht von hinten auf die Vorrichtung gemäß Figur 1,
- Fig. 3: eine perspektivische Ansicht eines weiteren Ausführungsbeispiels einer erfindungsgemäßen Vorrichtung,
- Fig. 4: eine Ansicht der Vorrichtung gemäß Fig. 3 in einem an eine Person angelegten Zustand und
- Fig. 5: eine Explosionsdarstellung der Vorrichtung gemäß Fig. 3.

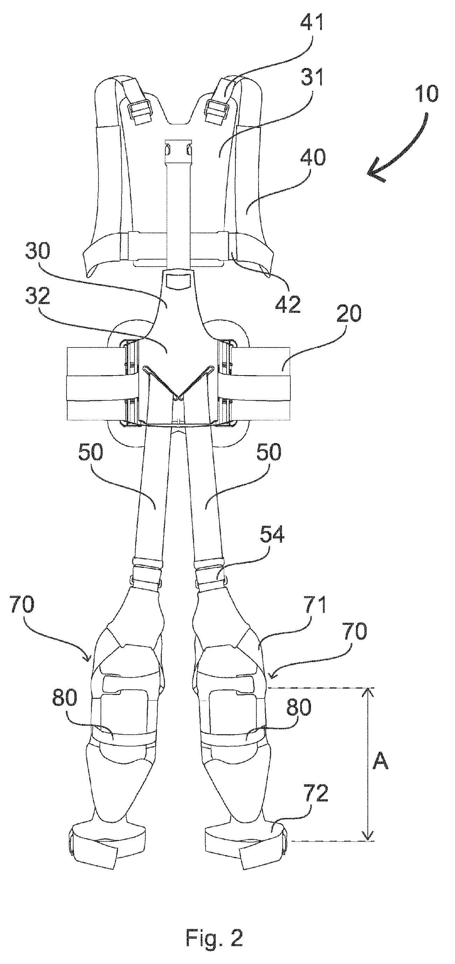

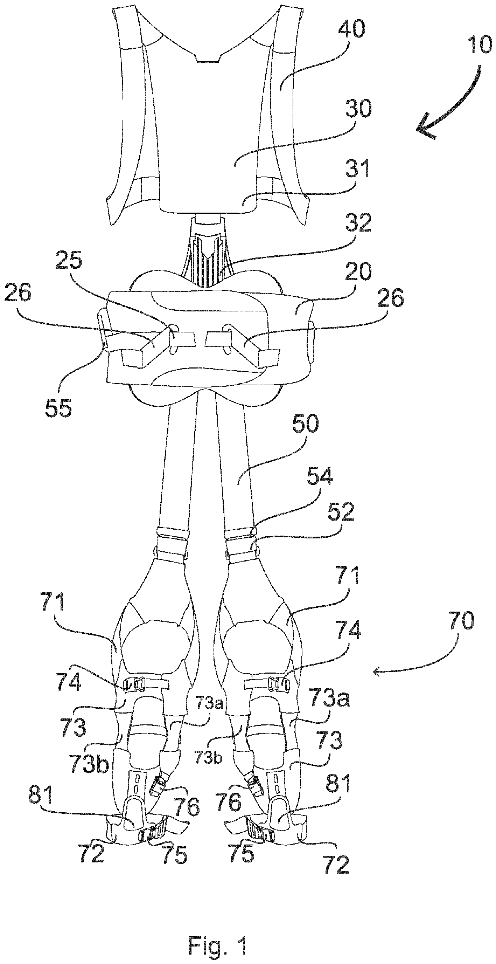

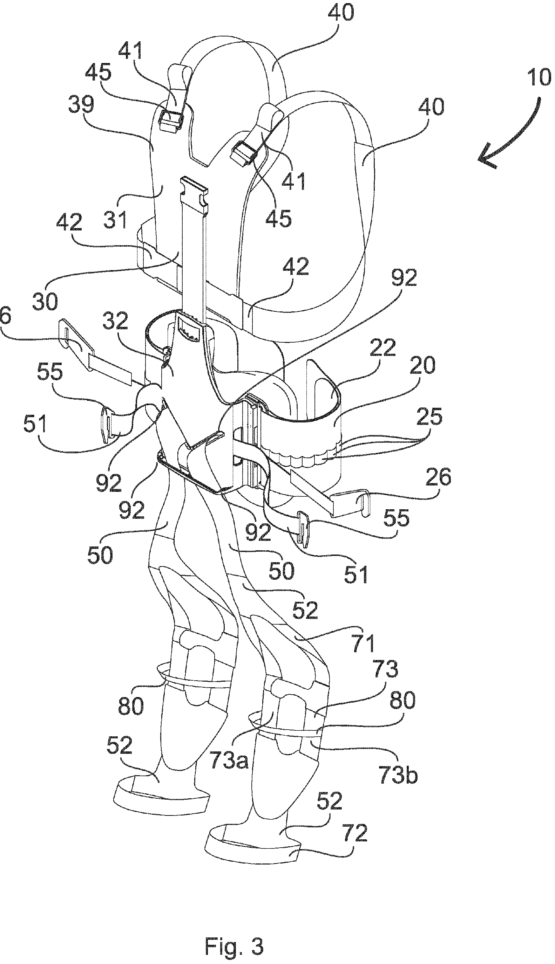

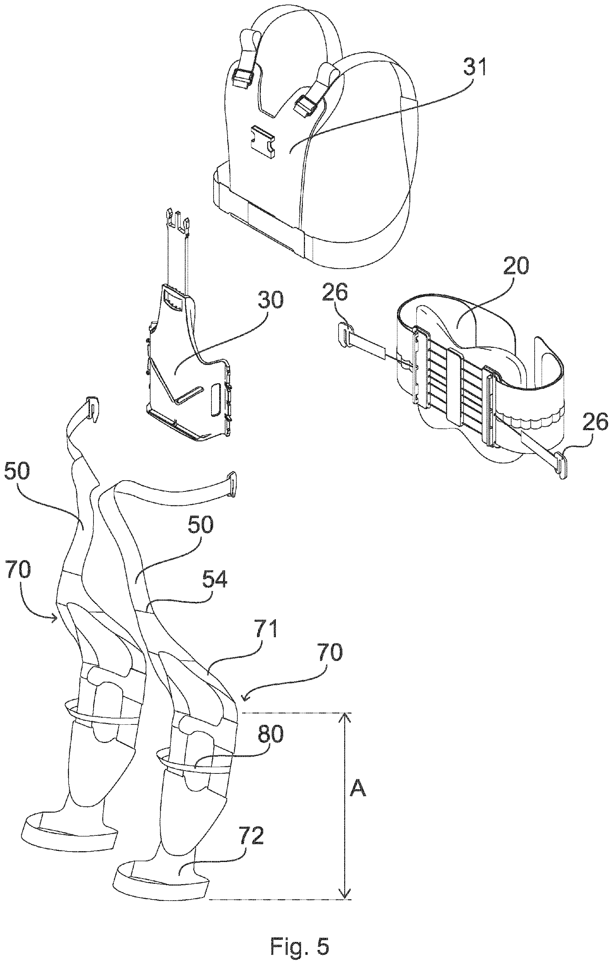

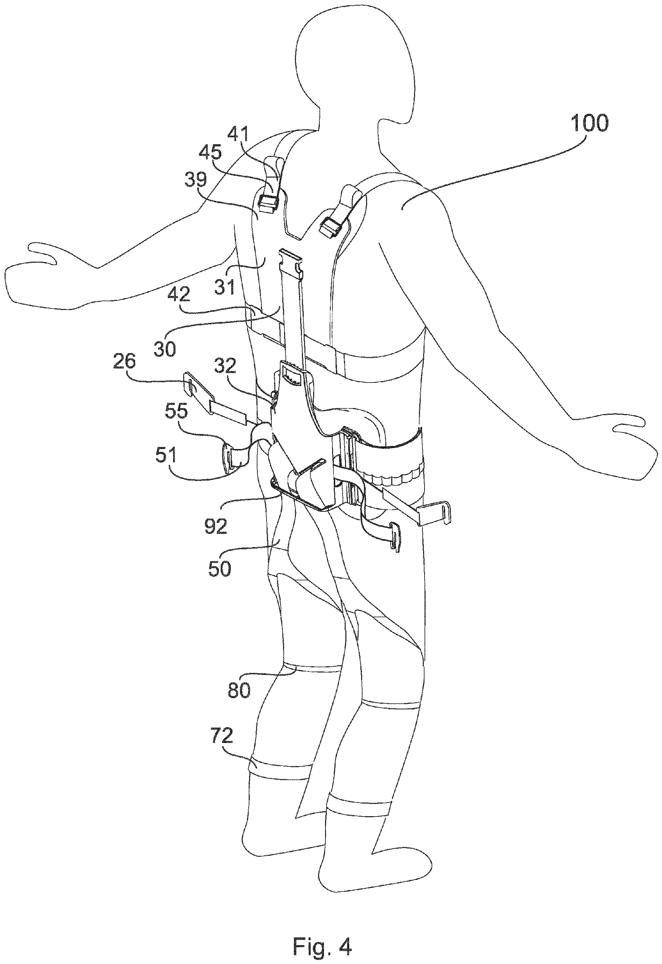

Die Figuren 1 und 2 zeigen verschiedene Ansichten eines ersten Ausführungsbeispiels einer Vorrichtung 10 zur Unterstützung von Bewegungen und Hebebewegungen des menschlichen Körpers, insbesondere eines passiven Exoskeletts. Die Figuren 3 und 5 zeigen eine perspektivische Ansicht sowie eine Explosionsdarstellung eines zweiten Ausführungsbeispiels einer Vorrichtung 10 zur Unterstützung von Bewegungen und Hebebewegungen des menschlichen Körpers, insbesondere eines passiven Exoskeletts, wobei Figur 4 die Vorrichtung 10 gemäß Figur 3 im an eine Person 100 angelegten Zustand darstellt. Die beiden Ausführungsbeispiele unterscheiden sich lediglich in Details, so dass die folgende Beschreibung, soweit nicht explizit angegeben, auf beide Ausführungsbeispiele zutrifft. Gleiche oder funktionsgleiche Teile sind in beiden Ausführungsbeispielen mit gleichen Bezugsziffern bezeichnet. Zur besseren Übersicht sind nicht sämtliche Bezugsziffern in sämtlichen Figuren angegeben.

Die Vorrichtung 10 weist ein Hüftelement 20 auf, welches in der vorliegenden Ausführungsform als Hüftgurt ausgebildet ist. Das Hüftelement 20 kann im Bereich der Hüfte an einer Person 100 fixiert werden, beispielsweise, indem es die Hüfte umgreift (vgl. Fig. 4). Zur Vereinfachung des Anlegens kann das Hüftelement 20 einen Verschluss nach Art eines Gürtels oder einen Klettverschluss 22 oder alternativ auch eine Schnappschnalle aufweisen. Dabei ist bei Verwendung einer Schnalle vorzugsweise wenigstens ein Ende des Hüftelements längenverstellbar an der Schnalle angeordnet, um die Weite des Hüftelements 20 verstellen zu können.

Das Hüftelement 20 kann aus einem textilen Material gefertigt sein.

An dem Hüftelement 20 sind zwei Beinbänder 50 angeordnet, insbesondere derart, dass sie im an die Person 100 angelegten Zustand der Vorrichtung ausgehend von der Hüfte über das Gesäß der Person 100 an den Beinen anliegend verlaufen (siehe Figur 4). Die Beinbänder 50 weisen ein erstes Ende 51 und ein zweites Ende 52 auf. Die Beinbänder 50 sind vorzugsweise lösbar mit dem ersten Ende 51 an dem Hüftelement 20 angeordnet, insbesondere an der Außenseite des Hüftelements 20, beispielsweise mittels eines Hakens 55, einer Schnappschnalle oder alternativ nach Art eines Verschluss eines Gürtels oder mittels eines Klettverschlusses oder sonstigen Verschlusses. An dem Hüftelement 20 können dafür insbesondere auf der Außenseite des Hüftelements 20 zumindest eine Tasche oder Schlaufe 25 gebildet sein, in welche der Haken 55 einhängbar ist, um die Beinbänder 50 lösbar befestigen zu können (vgl. Figur 1). Eine Längenverstellbarkeit der Beinbänder 50 kann durch eine insbesondere am zweiten Ende 52 des Beinbands 50 angeordnete Schnalle 54 ermöglicht werden. Alternativ können, wie bei dem in Figur 3 dargestellten Ausführungsbeispiel, mehrere Taschen oder Schlaufen 25 gebildet sein, in welche der Haken 55 einhängbar ist, um die Beinbänder 50 einerseits lösbar befestigen zu können und andererseits gleichzeitig eine Längenverstellbarkeit der Beinbänder 50 zu ermöglichen.

Um die Beinbänder 50 auf einfache Art und Weise seitlich an dem Hüftelement 20 befestigen zu können, können die Beinbänder 50 mit ihrem freien Ende 51 durch zwei im Winkel zueinander verlaufende Schlitze 92 geführt sein. Die Schlitze 92 können in dem Hüftelement 20 oder dem Rückelement 30 angeordnet sein. Die jeweils oberen Schlitze 92 sind insbesondere im Winkel von 45° gegen die Vertikale geneigt, die jeweils unteren Schlitze 92 verlaufen beispielsweise horizontal, bezogen auf den angelegten Zustand an einer stehenden Person 100 (vgl. insbesondere Figur 4). Dadurch kann eine Umlenkung des Beinbands 50 um 90° erreicht werden. Insbesondere sind die beiden jeweils oberen Schlitze 92 im Winkel von 90° zueinander verlaufend angeordnet.

An dem zweiten Ende 52 des Beinbands 50 ist ein Knieelement 70 angeordnet. Das Knieelement 70 weist eine obere Schlaufe 71, eine untere Schlaufe 72 und ein Kniescheibenelement 73 auf, wobei das Kniescheibenelement 73 derart ausgebildet ist, dass es ein Loch aufweist und insbesondere im an die Person 100 angelegten Zustand der Vorrichtung 10 die Kniescheibe umfassend ausgebildet ist. Wie in Figur 4 dargestellt, umgreift im an die Person 100 angelegten Zustand der Vorrichtung die obere Schlaufe 71 das Bein der Person 100 oberhalb des Knies, insbesondere im unteren Bereich des Oberschenkels. Die untere Schlaufe 72 umgreift im an die Person 100 angelegten Zustand der Vorrichtung 100 das Bein der Person 100 unterhalb des Knies, insbesondere unterhalb der Wade. Das Kniescheibenelement 73 verläuft mit zwei Längselementen 73a, 73b seitlich der Kniescheibe und lässt diese dabei vorzugsweise frei.

Die obere Schlaufe 71 und die untere Schlaufe 72 sind in einem Abstand A zueinander angeordnet, welcher verstellbar ausgebildet ist. Dazu kann die untere Schlaufe 72 lösbar an dem Kniescheibenelement 73 befestigbar sein. Insbesondere ist die untere Schlaufe 72 in unterschiedlichen Positionen an dem Kniescheibenelement 73 fixierbar. Beispielsweise ist die untere Schlaufe 72 mittels eines Verschlusses 81, der beispielsweise als Klettverschluss oder als Magnetverschluss oder als auf einer formschlüssigen Verbindung basierender Verschluss ausgebildet sein kann, lösbar an dem Kniescheibenelement 73 fixierbar und insbesondere in unterschiedlichen Positionen, die eine Variation des Abstands A erlauben, fixierbar. Über einen derartigen Verschluss 81 lässt sich die Fixierung in unterschiedlichen Position auf einfache Art und Weise realisieren.

An dem Kniescheibenelement 73 kann ein Befestigungselement 80 angeordnet sein, welches die beiden seitlichen Längselemente 73a, 73b verbindet und insbesondere im an die Person 100 angelegten Zustand über die Rückseite des Beins, insbesondere oberhalb der Wade, verläuft.

Das Kniescheibenelement 73 kann einen zusätzlichen Verschluss 76 aufweisen, mit welchem beispielsweise eines der Längselemente 73a, 73b öffenbar und wiederverschließbar ist, um das Anlegen des Knieelements 70 vereinfachen zu können. Der Verschluss 76 kann ebenfalls eine Längeneinstellbarkeit ermöglichen, beispielsweise, indem er als Schnalle ausgebildet ist, um eine möglichst optimale Anpassung an den Körper der Person 100 ermöglichen zu können.

Alternativ kann die untere Schlaufe 72 auf mittels eines Schienensystems verschiebbar und in unterschiedlichen Positionen fixierbar an dem Kniescheibenelement 73 angeordnet sein.

Die Beinbänder 50 sind zumindest abschnittsweise aus einem Elastomer gefertigt. Auch das Knieelement 70, insbesondere die Längselemente 73a, 73b, kann zumindest abschnittsweise aus einem Elastomer gefertigt sein. Das Elastomer kann insbesondere ein E-Modul von weniger als 600 N/mm², bevorzugt ein E-Modul von weniger als 150 N/mm², besonders bevorzugt ein E-Modul von weniger als 100 N/mm², vorzugsweise ein E-Modul von etwa 30 N/mm² aufweisen.

Das Knieelement 70 wird in einer Ausführungsform derart über den Fuß das Bein hinauf gestreift, bis die obere Schlaufe 71 im unteren Bereich des Oberschenkels und die untere Schlaufe 72 unterhalb der Wade anliegt. Dabei wird der Abstand A zwischen den beiden Schlaufen 71, 72 entsprechend angepasst. Alternativ ist es möglich, dass die obere Schlaufe 71 und/oder die untere Schlaufe 72 öffenbar und wiederverschließbar ausgebildet sind, beispielsweise mittels eines Klettverschlusses, eines auf einem Formschluss basierenden Verschlusses, eines Magnetverschlusses oder eines sonstigen Verschlusses 74, 75, wodurch ein Anlegen ohne Überstreifen ausgehend vom Fuß ermöglicht wird. Schließlich wird das Beinband 50 am Hüftelement 20 befestigt, beispielsweise mithilfe der Haken 55, wobei vorzugsweise die Länge des Beinbands 50 auf die Größe der Person 100 abgestimmt wird.

An dem Hüftelement 20 kann ein Rückenelement 30 angeordnet sein. Als Rückenelement 30 soll im Folgenden ein Element verstanden werden, dass zumindest abschnittsweise im an die Person 100 angelegten Zustand der Vorrichtung am Rücken der Person anliegt. Das Rückenelement 30 weist insbesondere eine derartige Länge auf, dass es im an die Person 100 angelegten Zustand vom Bereich der Hüfte bis in den Bereich des oberen Rückens oder Nackens erstreckt. Insbesondere kann das Rückenelement 30 auch an seinem dem Hüftelement 20 abgewandten Ende mit Abschnitten 39 auf den Schultern der Person 100 aufliegen. Das Rückenelement kann einstückig mit dem Hüftelement verbunden sein. Das Rückenelement 30 kann alternativ lösbar an dem Hüftelement 20 angeordnet, um einen modularen Aufbau zu ermöglichen.

Das Rückenelement 30 kann zumindest abschnittsweise, vorzugsweise vollständig, aus einem formelastischen Material gefertigt. Das formelastische Material kann beispielsweise Metall oder Kunststoff sein, wobei Kunststoff aufgrund des geringeren Gewichts in der Regel bevorzugt wird. Das formelastische Material weist vorzugsweise ein E-Modul von mehr als 600 N/mm², besonders bevorzugt ein E-Modul von mehr als 1000 N/mm², beispielsweise ein E-Modul von 5000 N/mm² auf.

Das Rückenelement 30 kann zweiteilig mit zwei Teilelementen 31, 32 ausgebildet sein, welche in der Länge verstellbar gegeneinander fixierbar sein können.

Die Vorrichtung 10 kann zwei Schulterbänder 40 aufweisen, welche jeweils ein erstens Ende 41 und ein zweites Ende 42 aufweisen. Als Schulterbänder 40 sollen im Folgenden die Elemente verstanden werden, die das Rückenelement 30 im an die Person 100 angelegten Zustand der Vorrichtung 10 am Rücken der Person anliegend fixieren. Sie können vom Rücken über die Schulter auf die Brust der Person 100 und unterhalb der Arm wieder zum Rücken geführt verlaufen. Es ist aber auch möglich, dass das Rückenelement 30 sowohl am Rücken als auch auf den Schultern aufliegt und die Schulterbänder 40 nicht auf der Schulter selbst, sondern im Wesentlichen auf der Brust der Person 100 verlaufen.

Das erste Ende 41 ist an dem Rückenelement 30, beispielsweise an den auf den Schultern der Person 100 aufliegenden Abschnitten 39 des Rückenelements 30, angeordnet. Das zweite Ende 42 kann an dem Hüftelement 20 oder dem Rückenelement 30 angeordnet sein und ist im vorliegenden Ausführungsbeispiel am Rückenelement 30 angeordnet. Die Schulterbänder 40 können mittels einer Schnalle 45 längenverstellbar an dem Rückenelement 30 angeordnet sein. Die Schulterbänder 40 können aus einem Gurtmaterial, beispielsweise aus einem Gewebeband, gefertigt sein.

Zum Anlegen der Vorrichtung 10 kann zunächst das Hüftelement 20 um die Hüfte gelegt werden. Falls vorhanden, werden die Verschlüsse 74, 75 der oberen und unteren Schlaufen 71, 72 geöffnet und die Schlaufen 71, 72 um das Bein gelegt. Dabei wird der Abstand A zwischen den Schlaufen 71, 72 an die Beinlänge angepasst, insbesondere, indem der Verschluss 81 geöffnet und in der gewünschten Position geschlossen bzw. fixiert wird. Nach Fixierung der Knieelemente 70 können die Beinbänder 50 in der gewünschten Länge an dem Hüftelement 20 befestigt werden, beispielsweise durch Einhängen der Haken 55 in die gewünschte Tasche oder Schlaufe 25.

Die Vorrichtung 10 kann weiter von der Person 100 angelegt werden, indem die Arme durch die durch das Rückenelement 30 und die Schulterbänder 40 gebildeten Schlaufen geführt werden, bis das Rückenelement 30 am Rücken anliegt. Dabei kann mithilfe der Schnallen 45 die gewünschte Länge der Schulterbänder 40 und die gewünschte Weite des Hüftelements 20 eingestellt werden, bis die Vorrichtung 10 eine Position erreicht hat, in welcher sie am Körper anliegt.

An dem Hüftelement 20 können Spannseile 26 angeordnet sein, welche es ermöglichen, das Hüftelement 20 nach dem Schließen zusätzlich zu spannen und dadurch zu festigen. Auch die Spannseile 26 können Haken aufweisen, durch welche sie in der wenigstens einen Tasche oder Schlaufe 25 eingehängt werden können.

Wie in Figur 5 dargestellt, ist die Vorrichtung 10 insbesondere modular aufgebaut und kann dadurch auf einfache Art und Weise zerlegt werden. Das Teilelement 32 des Rückenelements 30 kann über eine Steckverbindung mit dem Hüftelement 20 verbunden werden. Die beiden Teilelemente 31, 32 des Rückenelements können über eine Rastverbindung miteinander verbunden werden. Die Beinbänder 50 können ebenfalls aufgrund des freien Endes 51, welches lediglich mittels des Hakens 55 in die Taschen oder Schlaufen 25 eingehängt wird, einfach von dem Hüftelement 20 gelöst und mit diesem wieder verbunden werden. Eine derartige Modularisierung der Vorrichtung 10 ermöglicht eine einfache Trennung von waschbaren Teilen. Zudem können je nach Einsatzzweck die Module auch einzeln getragen werden.

### Bezugszeichenliste

- 10: Vorrichtung
- 20: Hüftelement
- 22: Verschluss
- 25: Schlaufe
- 30: Rückenelement
- 31: Teilelement
- 32: Teilelement
- 39: Abschnitt
- 40: Schulterband
- 41: erstes Ende
- 42: zweites Ende
- 45: Schnalle
- 50: Beinband
- 51: erstes Ende
- 52: zweites Ende
- 54: Schnalle
- 55: Haken
- 70: Knieelement
- 71: obere Schlaufe
- 72: untere Schlaufe
- 73: Kniescheibenelement
- 73a: Längselement
- 73b: Längselement
- 74: Verschluss
- 75: Verschluss
- 76: Verschluss
- 80: Befestigungselement
- 81: Verschluss
- 92: Schlitz
- A: Abstand

## Patentansprüche

1. Vorrichtung (10) zur Unterstützung von Bewegungen und Hebebewegungen des menschlichen Körpers, welche am Körper tragbar ist, mit einem Hüftelement (20) und mit zwei an dem Hüftelement (20) angeordneten Beinbändern (50), welche derart angeordnet sind, dass sie im an den Körper angelegten Zustand der Vorrichtung am Gesäß des Körpers entlang verlaufen, wobei jedes der Beinbänder (50) ein Knieelement (70) aufweist, welches eine obere Schlaufe (71), eine untere Schlaufe (72) und ein die Kniescheibe freilassendes Kniescheibenelement (73) aufweist,
**dadurch gekennzeichnet, dass** der Abstand (A) zwischen der oberen Schlaufe (71) und der unteren Schlaufe (72) verstellbar ausgebildet ist.

2. Vorrichtung nach einem der vorhergehenden Ansprüche,
**dadurch gekennzeichnet, dass** die untere Schlaufe (72) lösbar an dem Kniescheibenelement (73) befestigbar ist.

3. Vorrichtung nach einem der vorhergehenden Ansprüche,
**dadurch gekennzeichnet, dass** die untere Schlaufe (72) in unterschiedlichen Positionen an dem Kniescheibenelement (73) fixierbar ist.

4. Vorrichtung nach einem der vorhergehenden Ansprüche,
**dadurch gekennzeichnet, dass** die untere Schlaufe (72) mittels eines auf einer Klett- oder Magnetverbindung oder einer formschlüssigen Verbindung basierenden Verschlusses (81) an dem Kniescheibenelement (73) fixierbar ist.

5. Vorrichtung nach einem der vorhergehenden Ansprüche,
**dadurch gekennzeichnet, dass** die untere Schlaufe (72) mittels eines Schienensystems verschiebbar und in unterschiedlichen Positionen fixierbar an dem Kniescheibenelement (73) angeordnet ist.

6. Vorrichtung nach einem der vorhergehenden Ansprüche,
**dadurch gekennzeichnet, dass** die obere Schlaufe (71) und/oder die untere Schlaufe (72) mittels eines Verschlusses (74, 75) öffenbar und wiederverschließbar, beispielsweise mittels eines Klettverschlusses, eines auf einem Formschluss basierenden Verschlusses, eines Magnetverschlusses oder eines sonstigen Verschlusses, ausgebildet sind.

7. Vorrichtung nach einem der vorhergehenden Ansprüche,
**dadurch gekennzeichnet, dass** an dem Kniescheibenelement (73) ein Befestigungselement (80) angeordnet ist.

8. Vorrichtung nach einem der vorhergehenden Ansprüche,
**dadurch gekennzeichnet, dass** die Beinbänder (50) aus einem Textilmaterial gefertigt sind.

9. Vorrichtung nach einem der vorhergehenden Ansprüche,
**dadurch gekennzeichnet, dass** die Beinbänder (50) zumindest abschnittsweise aus einem Elastomer gefertigt sind, wobei das Elastomer vorzugsweise ein E-Modul von weniger als 600 N/mm² aufweist.

10. Vorrichtung nach einem der vorhergehenden Ansprüche,
**dadurch gekennzeichnet, dass** jedes der Beinbänder (50) lösbar an dem Hüftelement (20) befestigt ist.

11. Vorrichtung nach einem der vorhergehenden Ansprüche,
**dadurch gekennzeichnet, dass** jedes der Beinbänder (50) an der Außenseite des Hüftelements (20) befestigbar ist, beispielsweise mittels eines Hakens (52), einer Schnalle, einer Gürtelschnalle oder eines Klettverschlusses.

12. Vorrichtung nach einem der vorhergehenden Ansprüche,
**dadurch gekennzeichnet, dass** jedes der Beinbänder (50) mit einem freien Ende (51) durch zwei im Winkel, vorzugsweise im Winkel von 45°, zueinander verlaufende Schlitze (92), geführt ist.

13. Vorrichtung nach einem der vorhergehenden Ansprüche,
**dadurch gekennzeichnet, dass** jedes der Beinbänder (50) im Bereich zwischen dem Hüftelement (20) und dem Knieelement (70) in der Länge verstellbar ausgebildet ist.

## Claims

1. Device (10), which can be worn on the body, for supporting movements and lifting movements of the human body, having a waist element (20) and two leg bands (50) arranged on the waist element (20) which are arranged such that, when the device is worn on the body, they extend along the buttocks of the body, wherein each of the leg bands (50) comprises a knee element (70) which comprises an upper strap (71), a lower strap (72), and a knee cap element (73) which leaves the knee cap exposed, **characterized in that** the distance (A) between the upper strap (71) and the lower strap (72) is implemented to be adjustable.

2. Device in accordance with any of the preceding claims,
**characterized in that** the lower strap (72) is detachably attachable to the knee cap element (73).

3. Device in accordance with any of the preceding claims,
**characterized in that** the lower strap (72) can be secured in various positions on the knee cap element (73).

4. Device in accordance with any of the preceding claims,
**characterized in that** the lower strap (72) can be secured on the knee cap element (73) by means of a Velcro or magnet fastener or a fastener (81) based on a positive connection.

5. Device in accordance with any of the preceding claims,
**characterized in that** the lower strap (72) is moveably and securable in various positions arranged on the knee cap element (73) by means of a brace system.

6. Device in accordance with any of the preceding claims,
**characterized in that** the upper strap (71) and/or the lower strap (72) are implemented to be releasable and re-attachable by means of a fastener (74, 75), for example, by means of a Velcro fastener, a fastener based on a positive connection, a magnet fastener or other fastener.

7. Device in accordance with any of the preceding claims,
**characterized in that** a fastening element (80) is arranged on the knee cap element (73).

8. Device in accordance with any of the preceding claims,
**characterized in that** the leg bands (50) are manufactured of a textile material.

9. Device in accordance with any of the preceding claims,
**characterized in that** the leg bands (50) are at least partially manufactured of an elastomer, wherein the elastomer preferably comprises a modulus of elasticity of less than 600 N/mm².

10. Device in accordance with any of the preceding claims,
**characterized in that** each of the leg bands (50) is detachably attached to the waist element (20).

11. Device in accordance with any of the preceding claims,
**characterized in that** each of the leg bands (50) can be attached to the outside of the waist element (20), for example, by means of a hook (52), a clasp, a belt buckle or a Velcro fastener.

12. Device in accordance with any of the preceding claims,
**characterized in that** a free end (51) of each of the leg band (50) is inserted through two slits (92) which extend at an angle to each other, preferably at an angle of 45°.

13. Device in accordance with any of the preceding claims,
**characterized in that** each of the leg bands (50) is implemented to be adjustable in length in the region between the waist element (20) and the knee element (70).

## Revendications

1. Dispositif (10) d'aide aux mouvements et aux mouvements de soulèvement du corps humain, qui est porté par le corps, avec un élément de hanche (20) et deux bandes de jambes (50) sur l'élément de hanche (20), ces bandes étant disposées de façon que dans l'état du dispositif mis sur le corps, il passe au niveau du siège, le long du corps, chacune des bandes de jambe (50) ayant un élément de genou (70) qui comprend une boucle supérieure (71), une boucle inférieure (72) et un élément de rotule (73) laissant dégagée la rotule,
dispositif **caractérisé en ce que**
la distance (A) entre la boucle supérieure (71) et la boucle inférieure (72) est réglable.

2. Dispositif selon la revendication précédente,
**caractérisé en ce que**
la boucle inférieure (72) se fixe de manière amovible à l'élément de rotule (73).

3. Dispositif selon l'une des revendications précédentes,
**caractérisé en ce que**
la boucle inférieure (72) se fixe à l'élément de rotule (73) dans des positions différentes.

4. Dispositif selon l'une des revendications précédentes,
**caractérisé en ce que**
la boucle inférieure (72) se fixe avec une fermeture (81) à base de liaison à griffes ou de liaison magnétique ou de liaison par la forme à l'élément de rotule (73).

5. Dispositif selon l'une des revendications précédentes,
**caractérisé en ce que**
la boucle inférieure (72) est montée de manière coulissante par un système de rail et se fixe dans des positions différentes à l'élément de rotule (73).

6. Dispositif selon l'une des revendications précédentes,
**caractérisé en ce que**
la boucle supérieure (71) et/ou la boucle inférieure (72) s'ouvrent et se referment à l'aide d'un élément de fermeture (74, 75), par exemple, à l'aide d'une fermeture à griffes, d'une fermeture à liaison par la matière, d'une fermeture magnétique ou autre moyen de fermeture.

7. Dispositif selon l'une des revendications précédentes,
**caractérisé en ce que**
un élément de fixation (80) est prévu sur l'élément de rotule (73).

8. Dispositif selon l'une des revendications précédentes,
**caractérisé en ce que**
les bandes de jambe (50) sont en une matière textile.

9. Dispositif selon l'une des revendications précédentes,
**caractérisé en ce que**
les bandes de jambe (50) sont réalisées au moins par segments en une matière élastomère, la matière élastomère ayant de préférence un module d'élasticité E inférieur à 600 N/m².

10. Dispositif selon l'une des revendications précédentes,
**caractérisé en ce que**
chacune des bandes de jambe (50) se fixe de manière amovible à l'élément de hanche (20).

11. Dispositif selon l'une des revendications précédentes,
**caractérisé en ce que**
chacune des bandes de jambe (50) se fixe au côté extérieur de l'élément de hanche (20), par exemple, à l'aide d'un crochet (52), d'une boucle, d'une boucle de ceinture ou d'une fermeture à griffes.

12. Dispositif selon l'une des revendications précédentes,
**caractérisé en ce que**
chacune des bandes de jambe (50) passe par une extrémité libre (52) à travers deux fentes (92) faisant entre elles un angle, de préférence un angle de 45° par une extrémité libre (51).

13. Dispositif selon l'une des revendications précédentes,
**caractérisé en ce que**
chacune des bandes de jambe (50) est de longueur réglable dans la région entre l'élément de hanche (20) et l'élément de genou (70).
